# EUROPEAN PATENT APPLICATION

(11) **EP 4 509 085 A1**
(43) Date of publication of application: **19.02.2025**
(21) Application number: 24194812.4
(22) Date of filing: 15.08.2024
(51) Int. Cl.: A61B 34/30, A61B 1/00, A61B 1/06, A61B 1/313, A61B 1/04

(54) **SURGICAL ROBOTIC SYSTEM WITH AUTOMATED LOW VISIBILITY CONTROL**

(30) Priority: 16.08.2023 US 202363519873 P; 09.07.2024 US 202418766869
(71) Applicant: Covidien LP, Mansfield, MA 02048 (US)
(72) Inventor: STOROZUK, Nicole T., Warwick, 02886 (US); DENNIS, Andrew W., Lafayette, 80026 (US); DECKER, Ryan S., Boston, 02210 (US); DANIELS, Barret R., Boston, 02210 (US); BARSTEAD, Megan E., Boston, 02210 (US); EUSTON, Leslie E., Boston, 02210 (US); LUKASAK, Patrick L., Boston, 02210 (US)
(74) Representative: Maschio & Soames IP Ltd

(57) **Abstract**

A surgical robotic system includes an instrument drive unit coupled to and configured to actuate a surgical instrument and a laparoscopic camera. An image processing device is coupled to the laparoscopic camera and is configured to operate in a plurality of imaging modes having a low visibility mode. A surgeon console having a handle controller receives user input to move the surgical instrument. A controller operates the surgical instrument in a first operational mode while the low visibility mode is active, and in a second operational mode while the low visibility mode is not active.

## Description

### CROSS-REFERENCE TO RELATED APPLICATIONS

This application claims the benefit of, and priority to, U.S. Provisional Patent Application Serial No. 63/519,873 filed on August 16, 2023. The entire contents of the foregoing application is incorporated by reference herein.

### BACKGROUND

Surgical robotic systems are currently being used in a variety of surgical procedures, including minimally invasive surgical procedures. Some surgical robotic systems include a surgeon console controlling a surgical robotic arm and a surgical instrument having an end effector (e.g., forceps or grasping instrument) coupled to and actuated by the robotic arm. In operation, the robotic arm is moved to a position over a patient and then guides the surgical instrument into a small incision via a surgical port or a natural orifice of a patient to position the end effector at a work site within the patient's body. A laparoscopic camera, which is also held by one of the robotic arms, is inserted into the patient to image the surgical site.

The laparoscopic camera may operate in a variety of imaging modes including conventional color, or white light, mode and fluorescence mode. In conventional white light mode, light in the visible spectral range is used to illuminate the tissue surface under observation. Light reflected by the tissue passes through a suitable lens system and is incident on an image sensor built into or attached to the endoscope. The electrical signals from the image sensor are processed into a full color video image which can be displayed on a video monitor or stored in a memory.

In fluorescence mode, fluorescence excitation light excites fluorophores in the tissue, which emit fluorescence light at an emission wavelength, which is typically greater than the excitation wavelength. Fluorescence light from the tissue passes through a suitable lens system and is incident on the image sensor. The electrical signals from the image sensor are processed into a fluorescence video image which can be displayed on a video monitor, either separately or combined with the color video image.

The fluorescence excitation and emission wavelengths depend upon the type of fluorophores being excited. In the case of exogenously applied fluorophores, the band of excitation wavelengths may be located anywhere in the range from the ultraviolet (UV) to the near infra-red (NIR) and the emission wavelength band anywhere from the visible to the NIR. For fluorophores endogenous to tissue, the band of excitation and emission wavelengths are more limited (excitation from the UV to the green part of the visible spectrum, emission from the blue/green light to the NIR). Fluorescence imaging may be used to identify blood vessels, cancer cells, and other tissue types. White light and fluorescence imaging modes may be combined in a variety of ways. Camera manufacturers offer various imaging modes to provide surgeons additional insight into the structures and tools used during a laparoscopic or surgical procedures. Certain modes result in low visibility, during which instrument movement could lead to unexpected patient outcomes and inappropriate instrument usage. Similarly, using energy in a limited visibility mode could lead to adverse effects. Thus, there is a need for a system and method to control the surgical robotic systems in such low visibility modes.

### SUMMARY

The present disclosure provides a surgical robotic system that includes an imaging system that is operable in a plurality of imaging modes. The robotic system may include one or more robotic arms each holding an instrument or a laparoscopic camera of the imaging system. The imaging system is configured to obtain white color and NIR images of the tissue using fluorophores from a fluorescent dye, such as indocyanine green (ICG). The imaging system is configured to combine the white and NIR images in an overlay mode, during which the regular white light image is combined with the NIR/ICG data to generate an overlay image. In overlay mode, the imaging system may be configured to display the NIR image using visible light depending on user preferences and application, e.g., the NIR/ICG data can be displayed as a green or blue overlay. In intensity map mode, the imaging system displays the intensity of the NIR/ICG signal using a color scale in an overlay image. In a monochromatic mode, the NIR/ICG signal alone is displayed in white on a black background to achieve the greatest possible differentiation.

While in a mode that limits visibility of the cavity and/or instrument, such as monochromatic mode, the surgical system prevents the user from moving the instrument(s), the camera, or activating the instruments, e.g., delivering electrosurgical energy, ejecting staples, etc. Additionally, while controlling the instruments/camera, or while instrument function is selected, e.g., energy delivery or stapling, the system prevents the user from entering a low visibility mode.

Specific use cases include, but are not limited to, prevent energy selection while in a limited visibility mode, prevent position control while in a limited visibility mode, prevent entering limited visibility mode while energy is selected, prevent entering limited visibility mode while controlling instruments/camera, and provide notification to user if they attempt to perform one of the above to explain behavior.

Additional risk mitigation is also provided by the surgical robotic system. If the above conditions are not satisfied (e.g., major system malfunction has occurred), a system inoperable error is reported and certain functions of the instruments or the system as a whole are disabled. The imaging system may also switch to non-limited visibility mode, e.g., white color imaging, when user tries to access energy delivery or enter position control while in a limited visibility mode.

Specifically, while in the monochromatic mode, the system prevents position control and energy selection. If the user attempts to select energy or enter position control while in the monochromatic mode, the system ignores the user selection and provides the user a notification that position control and/or energy delivery is unavailable while in the monochrome mode.

While position control or while energy delivery is selected, the system prevents the user from selecting the monochromatic mode. Specifically, the monochromatic mode option is unavailable during this time, e.g., selection button is "greyed out" in a graphical user interface (GUI). If the user attempts to access monochromatic mode via the physical input (e.g., tapping a pedal controlling the camera), the input is ignored by the system and a notification is displayed to the user.

The surgical robotic system also includes various inputs e.g., foot pedals, GUI, etc., that may be used to switch between limited visibility and high visibility modes without looking away from screen. This allows the user to quickly switch between limited visibility and high visibility modes to utilize the limited visibility while also providing position control and energy delivery safely.

According to one embodiment of the present disclosure, a surgical robotic system is disclosed. The surgical robotic system includes an instrument drive unit coupled to and configured to actuate a surgical instrument and a laparoscopic camera. The system also includes an image processing device coupled to the laparoscopic camera and configured to operate in a plurality of imaging modes having a low visibility mode. The system further includes a surgeon console having a handle controller configured to receive user input to move the surgical instrument. The system additionally includes a controller configured to operate the surgical instrument in a first operational mode while the low visibility mode is active and in a second operational mode while the low visibility mode is not active.

Implementations of the above embodiment may include one or more of the following features. According to one aspect of the above embodiment, the laparoscopic camera is configured to capture white light and near infrared (NIR) light images. The low visibility mode may be a monochromatic NIR mode. The surgical robotic system may also include a display screen configured to display a video captured by the laparoscopic camera and processed by the image processing device based on a selected imaging mode of the plurality of imaging modes. While the surgical instrument is in the first operational mode, the controller may be configured to prevent activation of the surgical instrument. The activation of the surgical instrument may include one of supplying electrosurgical energy, ejecting staples, or advancing a knife. The controller may be further configured to output a prompt indicating activation of the surgical instrument was prevented. While in the first operational mode the controller may be additionally configured to switch from the low visibility mode to a default visibility imaging mode in response to the user input to move the surgical instrument.

According to another embodiment of the present disclosure, a method for controlling a surgical robotic system is disclosed. The method includes operating an image processing device coupled to a laparoscopic camera in a plurality of imaging modes, one of which is a low visibility mode. The method also includes receiving user input to move a surgical instrument at a surgeon console having a handle controller. The method further includes controlling the surgical instrument in a first operational mode while the low visibility mode is active. The method additionally includes controlling the surgical instrument in a second operational mode while the low visibility mode is not active.

Implementations of the above embodiment may include one or more of the following features. According to one aspect of the above embodiment, the method may include capturing white light and near infrared (NIR) light images at the laparoscopic camera. The low visibility mode may be a monochromatic NIR mode. The method may further include processing, by the image processing device, a video captured by the laparoscopic camera based on a selected imaging mode of the plurality of imaging modes and displaying the processed video on a display screen. The method may additionally include preventing activation of the surgical instrument while the surgical instrument is in the first operational mode. The activation of the surgical instrument may include supplying electrosurgical energy, ejecting staples, or advancing a knife. The method may also include displaying a prompt indicating activation of the surgical instrument was prevented. The method may further include automatically switching from the low visibility mode to a default visibility imaging mode in response to the user input to move the surgical instrument while the surgical instrument is in the first operational mode.

According to a further embodiment of the present disclosure, a method for controlling a surgical robotic system is disclosed. The method includes selecting an imaging mode for an image processing device from a plurality of imaging modes including a low visibility mode. The method also includes capturing images using a laparoscopic camera and processing the images at the image processing device using the selected imaging mode. The method further includes controlling a surgical robotic instrument in a first operational mode while the low visibility mode is active during which the surgical robotic instrument is prevented from being activated. The method additionally includes controlling the surgical robotic instrument in a second operational mode while the low visibility mode is not active, during which the surgical robotic instrument is movable and activatable to perform one or more functions.

Implementations of the above embodiment may include one or more of the following features. According to one aspect of the above embodiment, the method may also include receiving user input to move the surgical robotic instrument at a surgeon console may include a handle controller. The low visibility mode may be a monochromatic NIR mode. The function may be supplying electrosurgical energy, ejecting staples, or advancing a knife.

### BRIEF DESCRIPTION OF THE DRAWINGS

Various embodiments of the present disclosure are described herein with reference to the drawings wherein:
FIG. 1 is a perspective view of a surgical robotic system including a control tower, a console, and one or more surgical robotic arms each disposed on a mobile cart according to an embodiment of the present disclosure;
FIG. 2 is a perspective view of a surgical robotic arm of the surgical robotic system of FIG. 1 according to an embodiment of the present disclosure;
FIG. 3 is a perspective view of a mobile cart having a setup arm with the surgical robotic arm of the surgical robotic system of FIG. 1 according to an embodiment of the present disclosure;
FIG. 4 is a schematic diagram of a computer architecture of the surgical robotic system of FIG. 1 according to an embodiment of the present disclosure;
FIG. 5 is a plan schematic view of the surgical robotic system of FIG. 1 positioned about a surgical table according to an embodiment of the present disclosure;
FIG. 6 is a schematic diagram of a system for determining phases of a surgical procedure according to an embodiment of the present disclosure;
FIG. 7 is a perspective view of an imaging system according to an embodiment of the present disclosure;
FIG. 8 is a screenshot of a graphical user interface (GUI) for selecting an imaging mode according to an embodiment of the present disclosure;
FIG. 9 is a screenshot of a surgeon's display screen in a monochromatic imaging mode according to an embodiment of the present disclosure;
FIG. 10 is a screenshot of a surgeon's display screen in a color imaging mode according to an embodiment of the present disclosure; and
FIG. 11 is a flow chart of a method for automated low visibility control of the surgical robotic system of FIG. 1 according to an embodiment of the present disclosure.

### DETAILED DESCRIPTION

Embodiments of the presently disclosed surgical robotic system are described in detail with reference to the drawings, in which like reference numerals designate identical or corresponding elements in each of the several views. As used herein the term "coupled to" denotes a connection between components, which may be direct or indirect(i.e., through one or more components) and may be electronic, electrical, mechanical, or combinations thereof.

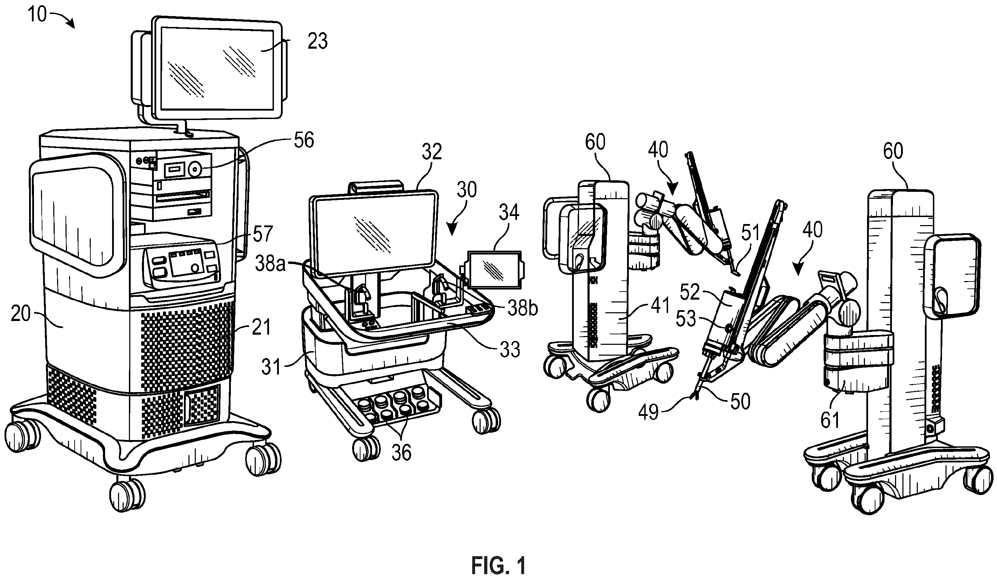

With reference to FIG. 1, a surgical robotic system 10 includes a control tower 20, which is connected to all the components of the surgical robotic system 10 including a surgeon console 30 and one or more mobile carts 60. Each of the mobile carts 60 includes a robotic arm 40 having a surgical instrument 50 removably coupled thereto. The robotic arms 40 also couple to the mobile carts 60. The robotic system 10 may include any number of mobile carts 60 and/or robotic arms 40.

The surgical instrument 50 is configured for use during minimally invasive surgical procedures. In embodiments, the surgical instrument 50 may be configured for open surgical procedures. In further embodiments, the surgical instrument 50 may be an electrosurgical or ultrasonic instrument, such as a forceps configured to seal tissue by compressing tissue between jaw members and applying electrosurgical current or ultrasonic vibrations via an ultrasonic transducer to the tissue. In yet further embodiments, the surgical instrument 50 may be a surgical stapler including a pair of jaws configured to grasp and clamp tissue while deploying a plurality of tissue fasteners, e.g., staples, and cutting stapled tissue. In yet further embodiments, the surgical instrument 50 may be a surgical clip applier including a pair of jaws configured apply a surgical clip onto tissue. The system also includes an electrosurgical generator 57 configured to output electrosurgical (e.g., monopolar or bipolar) or ultrasonic energy in a variety of operating modes, such as coagulation, cutting, sealing, etc. Suitable generators include a Valleylab^{™} FT10 Energy Platform available from Medtronic of Minneapolis, MN.

One of the robotic arms 40 may include a laparoscopic camera 51 configured to capture video of the surgical site. The laparoscopic camera 51 may be a stereoscopic camera configured to capture two side-by-side (i.e., left and right) images of the surgical site to produce a video stream of the surgical scene. The laparoscopic camera 51 is coupled to an image processing device 56, which may be disposed within the control tower 20. The image processing device 56 may be any computing device configured to receive the video feed from the laparoscopic camera 51 and output the processed video stream.

The surgeon console 30 includes a first, i.e., surgeon, screen 32, which displays a video feed of the surgical site provided by camera 51 of the surgical instrument 50 disposed on the robotic arm 40, and a second screen 34, which displays a user interface for controlling the surgical robotic system 10. The first screen 32 and second screen 34 may be touchscreens allowing for displaying various graphical user inputs.

The surgeon console 30 also includes a plurality of user interface devices, such as foot pedals 36 and a pair of hand controllers 38a and 38b which are used by a user to remotely control robotic arms 40. The surgeon console further includes an armrest 33 used to support clinician's arms while operating the hand controllers 38a and 38b.

The control tower 20 includes a screen 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instrument 50 execute a desired movement sequence in response to input from the foot pedals 36 and the hand controllers 38a and 38b. The foot pedals 36 may be used to enable and lock the hand controllers 38a and 38b, repositioning camera movement and electrosurgical activation/deactivation. In particular, the foot pedals 36 may be used to perform a clutching action on the hand controllers 38a and 38b. Clutching is initiated by pressing one of the foot pedals 36, which disconnects (i.e., prevents movement inputs) the hand controllers 38a and/or 38b from the robotic arm 40 and corresponding instrument 50 or camera 51 attached thereto. This allows the user to reposition the hand controllers 38a and 38b without moving the robotic arm(s) 40 and the instrument 50 and/or camera 51. This is useful when reaching control boundaries of the surgical space.

Each of the control tower 20, the surgeon console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area network, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DC). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-1203 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

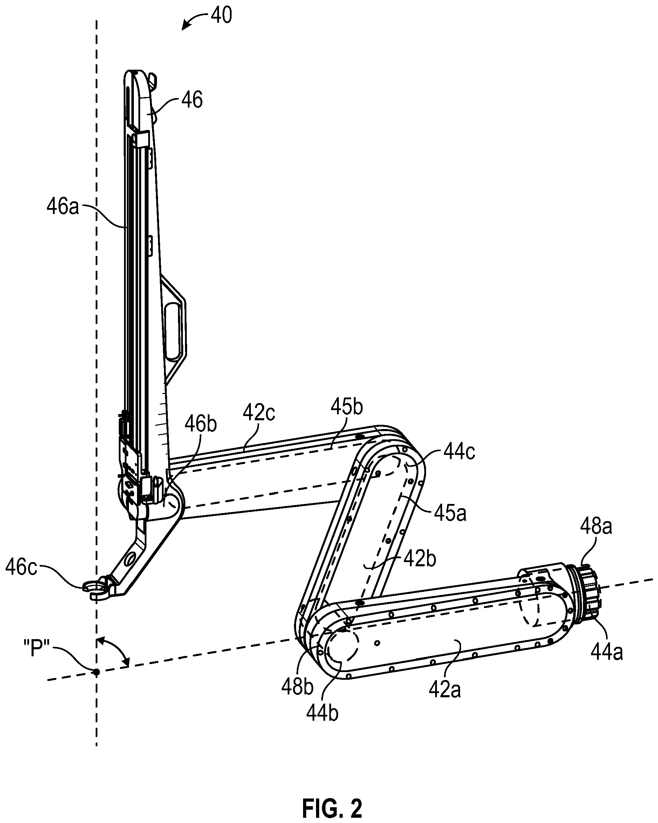

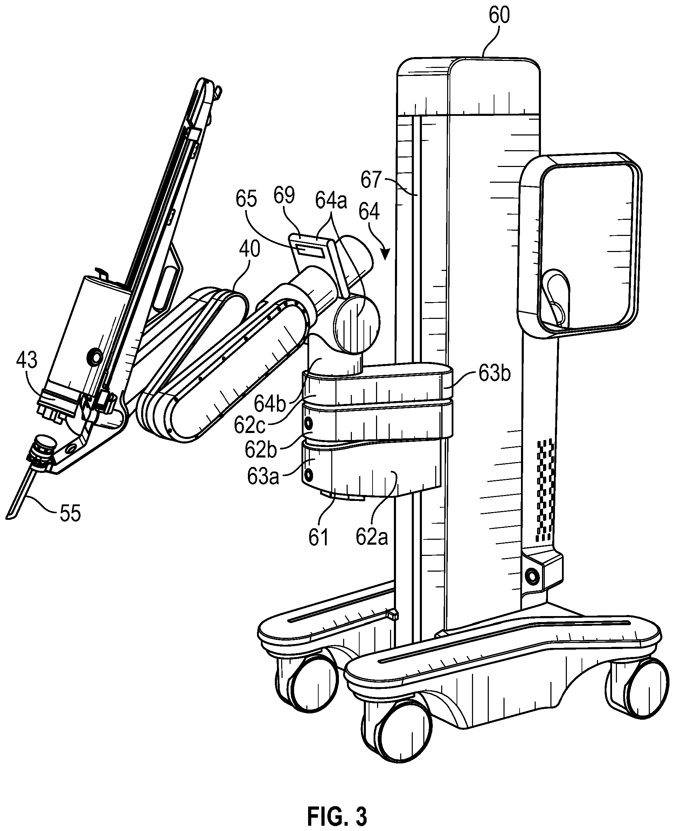

With reference to FIG. 2, each of the robotic arms 40 may include a plurality of links 42a, 42b, 42c, which are interconnected at joints 44a, 44b, 44c, respectively. Other configurations of links and joints may be utilized as known by those skilled in the art. The joint 44a is configured to secure the robotic arm 40 to the mobile cart 60 and defines a first longitudinal axis. With reference to FIG. 3, the mobile cart 60 includes a lift 67 and a setup arm 61, which provides a base for mounting the robotic arm 40. The lift 67 allows for vertical movement of the setup arm 61. The mobile cart 60 also includes a screen 69 for displaying information pertaining to the robotic arm 40. In embodiments, the robotic arm 40 may include any type and/or number of joints.

The setup arm 61 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 61 includes controls 65 for adjusting movement of the links 62a, 62b, 62c as well as the lift 67. In embodiments, the setup arm 61 may include any type and/or number of joints.

The third link 62c may include a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46b via the belt 45b. Joint 44c may include a transfer case coupling the belts 45a and 45b, such that the actuator 48b is configured to rotate each of the links 42b, 42c and a holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. In other words, the pivot point "P" is a remote center of motion (RCM) for the robotic arm 40. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted to achieve the desired angle θ. In embodiments, some or all of the joints 44a, 44b, 44c may include an actuator to obviate the need for mechanical linkages.

The j oints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the holder 46 defines a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components an end effector 49 of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During laparoscopic procedures, the instrument 50 may be inserted through a laparoscopic access port 55 (FIG. 3) held by the holder 46. The holder 46 also includes a port latch 46c for securing the access port 55 to the holder 46 (FIG. 2).

The robotic arm 40 also includes a plurality of manual override buttons 53 (FIG. 1) disposed on the IDU 52 and the setup arm 61, which may be used in a manual mode. The user may press one or more of the buttons 53 to move the component associated with the button 53.

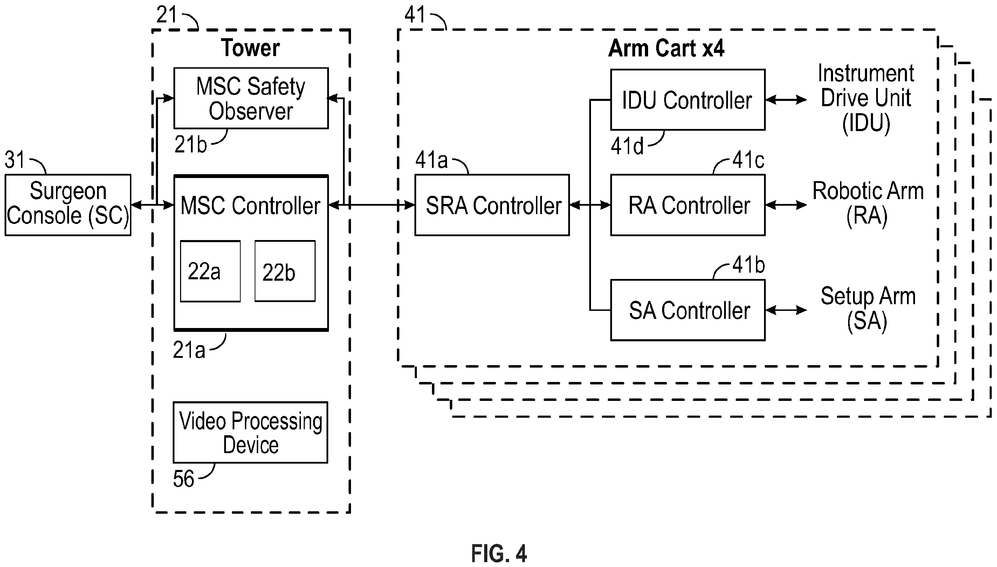

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgeon console 30 about the current position and/or orientation of the hand controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgeon console 30 to provide haptic feedback through the hand controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The controller 21a is coupled to a storage 22a, which may be non-transitory computer-readable medium configured to store any suitable computer data, such as software instructions executable by the controller 21a. The controller 21a also includes transitory memory 22b for loading instructions and other computer readable data during execution of the instructions. In embodiments, other controllers of the system 10 include similar configurations.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the mobile cart 60, the robotic arm 40, and the IDU 52. The main cart controller 41a also communicates actual joint angles back to the controller 21a.

Each of joints 63a and 63b and the rotatable base 64 of the setup arm 61 are passive joints (i.e., no actuators are present therein) allowing for manual adjustment thereof by a user. The joints 63a and 63b and the rotatable base 64 include brakes that are disengaged by the user to configure the setup arm 61. The setup arm controller 41b monitors slippage of each of joints 63a and 63b and the rotatable base 64 of the setup arm 61, when brakes are engaged or can be freely moved by the operator when brakes are disengaged, but do not impact controls of other joints. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

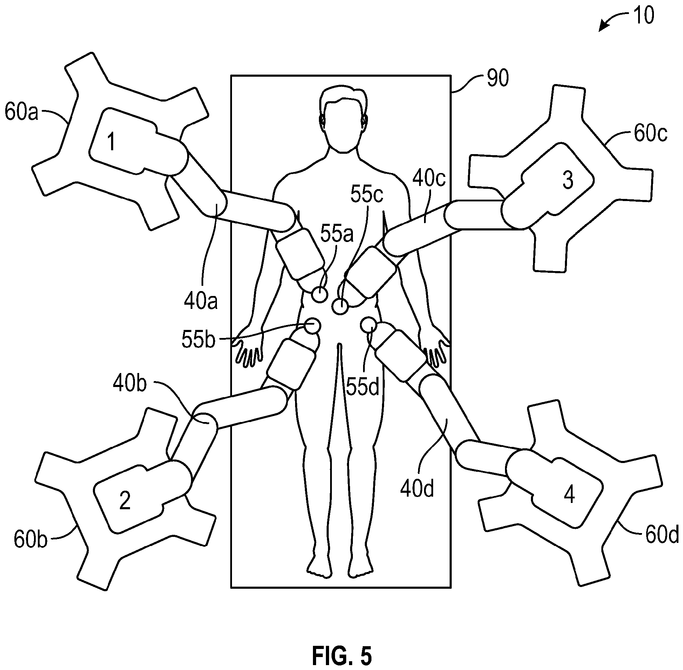

With reference to FIG. 5, the surgical robotic system 10 is set up around a surgical table 90. The system 10 includes mobile carts 60a-d, which may be numbered "1" through "4." During setup, each of the carts 60a-d are positioned around the surgical table 90. Position and orientation of the carts 60a-d depends on a plurality of factors, such as placement of a plurality of access ports 55a-d, which in turn, depends on the surgery being performed. Once the port placement is determined, the access ports 55a-d are inserted into the patient, and carts 60a-d are positioned to insert instruments 50 and the laparoscopic camera 51 into corresponding ports 55a-d.

During use, each of the robotic arms 40a-d is attached to one of the access ports 55a-d that is inserted into the patient by attaching the latch 46c (FIG. 2) to the access port 55 (FIG. 3). The IDU 52 is attached to the holder 46, followed by the SIM 43 being attached to a distal portion of the IDU 52. Thereafter, the instrument 50 is attached to the SIM 43. The instrument 50 is then inserted through the access port 55 by moving the IDU 52 along the holder 46. The SIM 43 includes a plurality of drive shafts configured to transmit rotation of individual motors of the IDU 52 to the instrument 50 thereby actuating the instrument 50. In addition, the SIM 43 provides a sterile barrier between the instrument 50 and the other components of robotic arm 40, including the IDU 52. The SIM 43 is also configured to secure a sterile drape (not shown) to the IDU 52.

A surgical procedure may include multiple phases, and each phase may include one or more surgical actions. As used herein, the term "phase" represents a surgical event that is composed of a series of steps (e.g., closure). A "surgical action" may include an incision, a compression, a stapling, a clipping, a suturing, a cauterization, a sealing, or any other such actions performed to complete a phase in the surgical procedure. A "step" refers to the completion of a named surgical objective (e.g., hemostasis). During each step, certain surgical instruments 50 (e.g., forceps) are used to achieve a specific objective by performing one or more surgical actions.

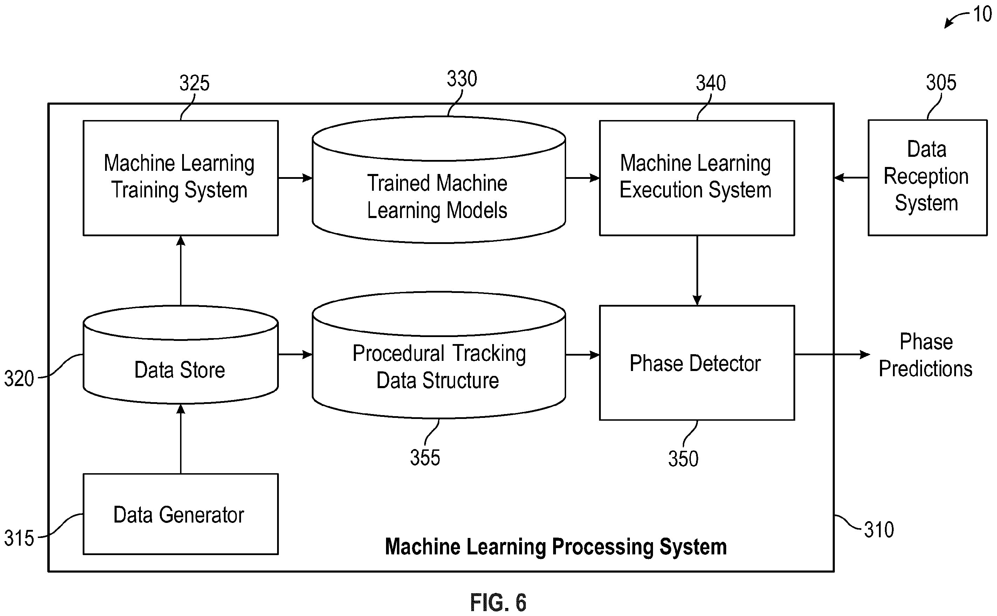

With reference to FIG. 6, the surgical robotic system 10 may include a machine learning (ML) processing system 310 that processes the surgical data using one or more ML models to identify one or more features, such as surgical phase, instrument, anatomical structure, etc., in the surgical data. The ML processing system 310 includes a ML training system 325, which may be a separate device (e.g., server) that stores its output as one or more trained ML models 330. The ML models 330 are accessible by a ML execution system 340. The ML execution system 340 may be separate from the ML training system 325, namely, devices that "train" the models are separate from devices that "infer," i.e., perform real-time processing of surgical data using the trained ML models 330.

System 10 includes a data reception system 305 that collects surgical data, including the video data and surgical instrumentation data. The data reception system 305 can include one or more devices (e.g., one or more user devices and/or servers) located within and/or associated with a surgical operating room and/or control center. The data reception system 305 can receive surgical data in real-time, i.e., as the surgical procedure is being performed.

The ML processing system 310, in some examples, may further include a data generator 315 to generate simulated surgical data, such as a set of virtual images, or record the video data from the image processing device 56, to train the ML models 330 as well as other sources of data, e.g., user input, arm movement, etc. Data generator 315 can access (read/write) a data store 320 to record data, including multiple images and/or multiple videos.

The ML processing system 310 also includes a phase detector 350 that uses the ML models to identify a phase within the surgical procedure. Phase detector 350 uses a particular procedural tracking data structure 355 from a list of procedural tracking data structures. Phase detector 350 selects the procedural tracking data structure 355 based on the type of surgical procedure that is being performed. In one or more examples, the type of surgical procedure is predetermined or input by user. The procedural tracking data structure 355 identifies a set of potential phases that may correspond to a part of the specific type of surgical procedure.

In some examples, the procedural tracking data structure 355 may be a graph that includes a set of nodes and a set of edges, with each node corresponding to a potential phase. The edges may provide directional connections between nodes that indicate (via the direction) an expected order during which the phases will be encountered throughout an iteration of the surgical procedure. The procedural tracking data structure 355 may include one or more branching nodes that feed to multiple next nodes and/or may include one or more points of divergence and/or convergence between the nodes. In some instances, a phase indicates a procedural action (e.g., surgical action) that is being performed or has been performed and/or indicates a combination of actions that have been performed. In some instances, a phase relates to a biological state of a patient undergoing a surgical procedure. For example, the biological state may indicate a complication (e.g., blood clots, clogged arteries/veins, etc.), pre-condition (e.g., lesions, polyps, etc.). In some examples, the ML models 330 are trained to detect an "abnormal condition," such as hemorrhaging, arrhythmias, blood vessel abnormality, etc.

The phase detector 350 outputs the phase prediction associated with a portion of the video data that is analyzed by the ML processing system 310. The phase prediction is associated with the portion of the video data by identifying a start time and an end time of the portion of the video that is analyzed by the ML execution system 340. The phase prediction that is output may include an identity of a surgical phase as detected by the phase detector 350 based on the output of the ML execution system 340. Further, the phase prediction, in one or more examples, may include identities of the structures (e.g., instrument, anatomy, etc.) that are identified by the ML execution system 340 in the portion of the video that is analyzed. The phase prediction may also include a confidence score of the prediction. Other examples may include various other types of information in the phase prediction that is output. The predicted phase may be used by the controller 21a to determine when to switch between various visibility modes as described below.

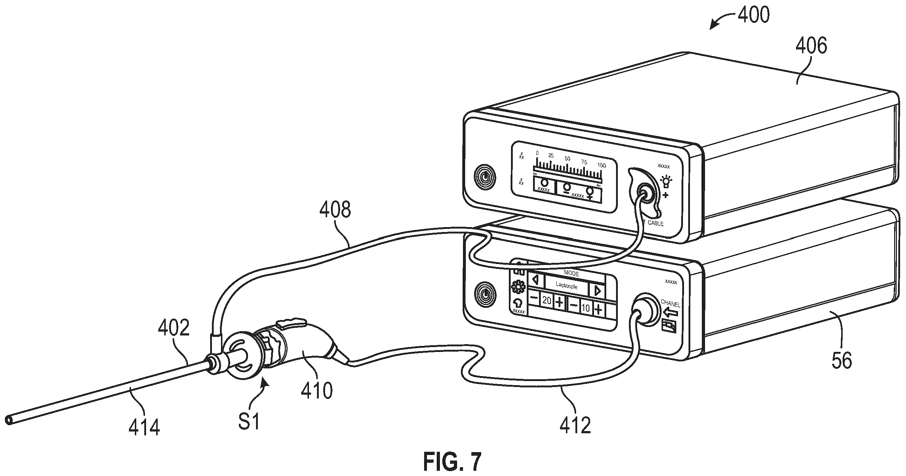

With reference to FIG. 7, the surgical robotic system 10 also includes an imaging system 400, in which the laparoscopic camera 51 coupled to the image processing device 56. The laparoscopic camera 51 includes a laparoscope 402 having a longitudinal shaft 414 with a plurality of optical components (not shown), such as lenses, mirrors, prisms, and the like disposed in the longitudinal shaft 414. The laparoscope 402 is coupled to a combined light source 406 via an optical cable 408. The light source 406 may include a white light source (not shown) and an NIR light source (not shown), which may be light emitting diodes or any other suitable light sources. The NIR light source may be a laser or any other suitable light source. The optical cable 408 may include one or more optical fibers for transmitting the white and NIR light, which illuminates the tissue under observation by the laparoscope 402. The laparoscope 402 collects the reflected white and NIR light and transmits the same to a camera assembly 410, which is coupled to a proximal end portion of the laparoscope 402. The laparoscope 402 may be any conventional laparoscopic configured to transmit and collect white and NIR light.

The camera assembly 410 includes is configured to separate fluorescence wavelength from undesired components of the light spectrum to specific sensors. In particular, the camera assembly includes a white (e.g., visible) light (VIS) sensor and an IR sensor and is configured to separate and transmit white light to the VIS sensor and fluorescence IR light to the IR sensor. The VIS sensor and the IR sensor may be a complementary metal oxide semiconductor (CMOS) image sensors having any desired resolution, which in embodiments may be 4K, UHD, etc.

The camera assembly 410 is coupled to the image processing device 56 via a transmission cable 412. The image processing device 56 is configured to receive the image data signals, process the raw image data from the camera assembly 410, and generate blended white light and NIR images for recording and/or real-time display. The image processing device 56 also processes the image data signals and outputs the same to any of the display screens 23, 32, 34 of the surgical robotic system 10, through any suitable a video output port, such as a DISPLAYPORT^{™}, HDMI^{®}, etc., that is capable of transmitting processed images at any desired resolution, display rates, and/or bandwidth.

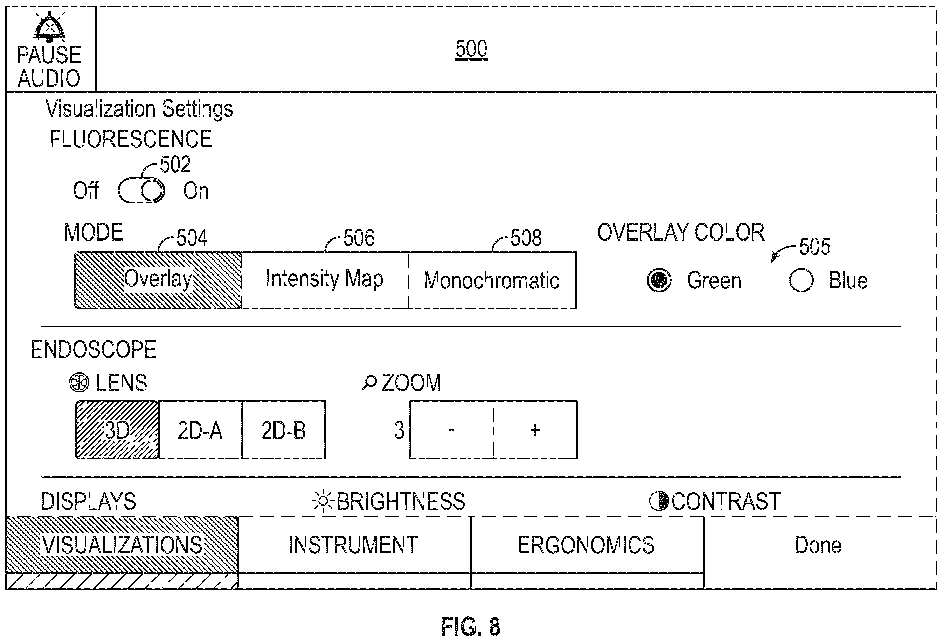

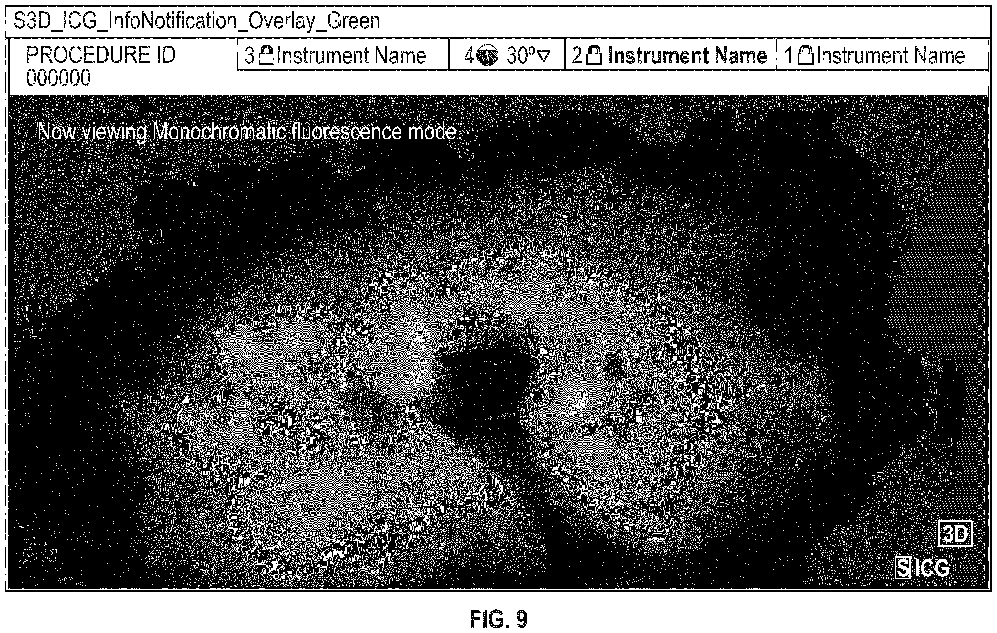

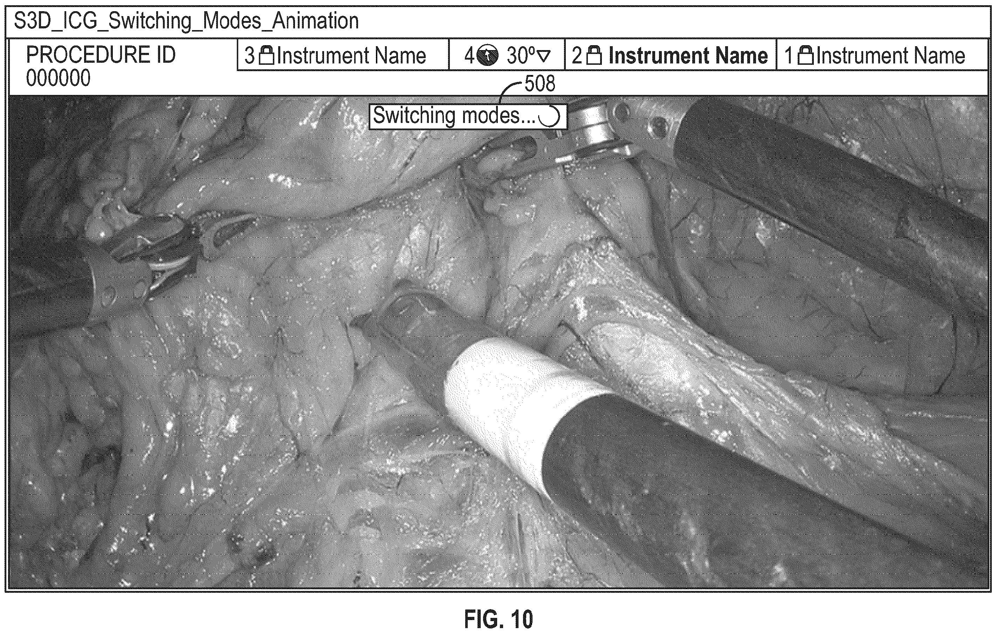

FIG. 8 shows a GUI 500 for controlling the imaging system 400, which may be displayed on the display screens 23, 32, 34 of the surgical robotic system 10. The GUI 500 includes options for controlling fluorescence settings, including turning fluorescence (e.g., NIR detection) on or off via toggle 502. Once fluorescence is selected, the user may also select from a plurality of imaging modes that visualize NIR light. An overlay mode is selectable via a button 504. In overlay mode, the imaging system 400 combines the white and NIR images, during which the regular white light image is combined with the NIR/ICG data to generate an overlay image. In overlay mode, the imaging system may be configured to display the NIR light in a visible light depending on user preferences and application. The NIR/ICG data can be displayed as a green or blue overlay, which is selectable via a menu 505. In intensity map mode, selectable via button 506, the imaging system displays the intensity of the NIR/ICG signal using a color scale in an overlay image. In a monochromatic mode, selectable via button 508, the NIR/ICG signal alone is displayed in white on a black background to achieve the greatest possible differentiation as shown in FIG. 9. In embodiments, the modes may be also selectable via one or more foot pedals 36 associated with mode selection, e.g., one foot pedal cycles through each of the NIR modes. FIG. 10 shows a white light video feed displayed on the first screen 32 from the camera 51 as well as an indicator that the imaging system 400 is switching between imaging modes.

The imaging system 400 may also provide marking capability to allow the user to mark landmarks on the video so that the surgeon can use limited visibility mode to identify landmarks, switch to a high visibility mode to move instruments and delivery energy and use the marker from the limited visibility mode. The marker may be removed from the screen if the camera moves so that the marker is not applied to a different portion of the interior. Alternatively, artificial intelligence or machine learning (AI/ML) computer vision algorithms may be used to identify the landmark and allow the landmark to transition with the camera.

The user or surgical robotic system may generate a so-called "bookmark" for one or more camera positions, which are then selectable by the user to return to any of the bookmarks. The imaging system also provides the user to take screenshots in the limited visibility view to allow the user to compare the limited visibility view while in a high visibility view alongside on one or more displays on a surgeon console.

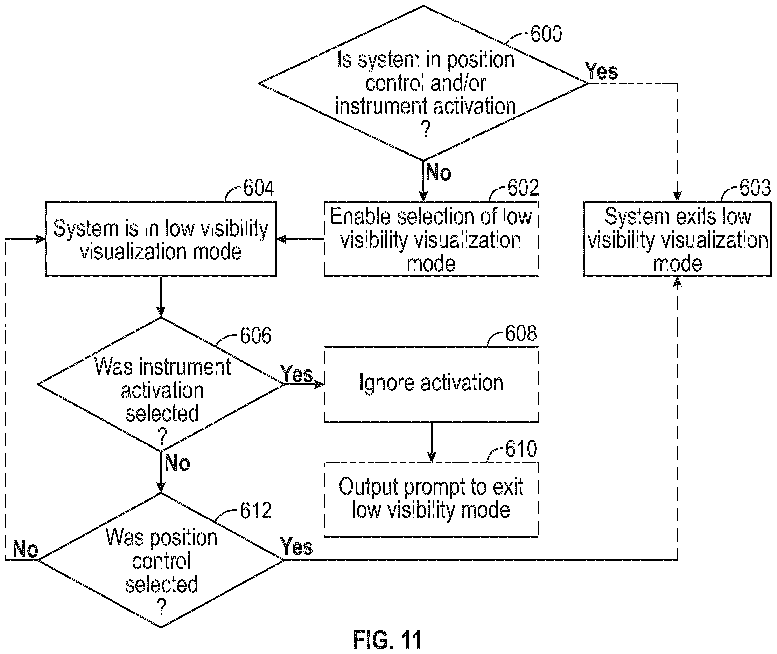

FIG. 11 shows a method of operating the surgical robotic system 10 along with the imaging system 400, where selected imaging modes are affected by the operational modes of the robotic arms 40a-d and vice versa. The method of FIG. 11 may be embodied as software instructions executable by any controller of the system 10, e.g., main controller 21a. At step 600, the controller 21a determines whether the system 10 is in position control and/or the instrument 50 is being activated. As used herein, position control includes any movement commands input at the handle controller 38a and 38b or other input devices to move the robotic arms 40a-d and their attachments, e.g., instrument 50, camera 51, etc. Activation of the instrument 50, which is different from movement, includes enabling or toggling electrosurgical energy delivery or another function of the instrument 50, e.g., to eject staples, advance a knife to cut tissue etc. If the system 10 is in either of these operational modes, then the imaging system 400 is in any high imaging mode, which may be the white light mode, or any of the combined white light/NIR modes described above, e.g., overlay or heat map modes.

If the system 10 is neither of these operational modes, then at step 602 the controller 21a enables imaging mode selection. At step 604, the user may select a desired imaging mode, including a low visibility mode, such as monochromatic mode, to observe the surgical site. In embodiments, imaging mode selection may be done automatically based on a specific phase of the surgical procedure as provided by the phase detector 350.

Once in the low visibility mode, the controller 21a operates in a first operational mode and performs verifications of user input. The first one is done at step 606, during which the controller 21a determines whether the user attempts to activate the instrument 50 by monitoring user input, e.g., pressing a button on the handle controllers 38a and 38b or one of the foot pedals 36 to energize the instrument 50 via the generator 57. If activation input is detected, at step 608 the controller 21a ignores the command while the low visibility mode is active. At step 610 the controller 21a also provides an indication of that the input just received was not executed due to the current imaging mode. The indication may be a prompt on the GUI 500 shown on any of the display screens 23, 32, 34.

In addition to limiting activation of the instrument 50, the controller 21a also reacts to movement inputs for position control of the instrument 50 and/or the camera 51. At step 612, the controller 21a determines whether the user attempts to move the instrument 50 and/or the camera 51 by monitoring user input, e.g., movement of the handle controllers 38a and 38b, while the low visibility mode is active. If no movement is detected, the controller 21a keeps the imaging system 400 in the selected low visibility mode, e.g., monochromatic imaging mode, and returns to step 604. If movement is detected, the controller 21a automatically exits the low visibility mode and enters a default or normal imaging mode at step 603, which may be the white light mode, or any of the combined white light/NIR modes described above, e.g., overlay or heat map modes. The user may also select to switch to one of these imaging modes as well. In embodiments, the switch may be automatic based on the detected phase of the surgical procedure. While the default imaging mode is active, the controller 21a operates the surgical robotic system 10 in a second, i.e., normal, operational mode, without restricting user input with respect to activating or moving instrument 50 and/or the camera 51.

It will be understood that various modifications may be made to the embodiments disclosed herein. Therefore, the above description should not be construed as limiting, but merely as exemplifications of various embodiments. Those skilled in the art will envision other modifications within the scope and spirit of the claims appended thereto.

The invention may be described by reference to the following numbered paragraphs:-
1. A surgical robotic system comprising:
   an instrument drive unit;
   a surgical instrument actuated by the instrument drive unit;
   a laparoscopic camera;
   an image processing device coupled to the laparoscopic camera and operable in a plurality of imaging modes including a low visibility mode;
   a surgeon console including a handle controller receiving user input to move the surgical instrument; and
   a controller operating the surgical instrument in a first operational mode while the low visibility mode is active and in a second operational mode while the low visibility mode is not active.
2. The surgical robotic system according to paragraph 1, wherein the laparoscopic camera is configured to capture white light and near infrared (NIR) light images.
3. The surgical robotic system according to paragraph 2, wherein the low visibility mode is a monochromatic NIR mode.
4. The surgical robotic system according to paragraph 1, further comprising:
   a display screen configured to display a video captured by the laparoscopic camera and processed by the image processing device based on a selected imaging mode of the plurality of imaging modes.
5. The surgical robotic system according to paragraph 4, wherein while the surgical instrument is in the first operational mode the controller is configured to prevent activation of the surgical instrument.
6. The surgical robotic system according to paragraph 5, wherein the activation of the surgical instrument includes at least one of supplying electrosurgical energy, ejecting staples, or advancing a knife.
7. The surgical robotic system according to paragraph 5, wherein the controller is further configured to output a prompt indicating activation of the surgical instrument was prevented.
8. The surgical robotic system according to paragraph 1, wherein while the surgical instrument is in the first operational mode, the controller is configured to switch from the low visibility mode to a default visibility imaging mode in response to a user input to move the surgical instrument.
9. A method for controlling a surgical robotic system, the method comprising:
   operating an image processing device coupled to a laparoscopic camera in a plurality of imaging modes including a low visibility mode;
   receiving user input to move a surgical instrument at a surgeon console including a handle controller;
   controlling the surgical instrument in a first operational mode while the low visibility mode is active; and
   controlling the surgical instrument in a second operational mode while the low visibility mode is not active.
10. The method according to paragraph 9, further comprising:
   capturing white light and near infrared (NIR) light images at the laparoscopic camera.
11. The method according to paragraph 10, wherein the low visibility mode is a monochromatic NIR mode.
12. The method according to paragraph 9, further comprising:
   processing, by the image processing device, a video captured by the laparoscopic camera based on a selected imaging mode of the plurality of imaging modes; and
   displaying the processed video on a display screen.
13. The method according to paragraph 12, further comprising:
   preventing activation of the surgical instrument while the surgical instrument is in the first operational mode.
14. The method according to paragraph 13, wherein the activation of the surgical instrument includes at least one of supplying electrosurgical energy, ejecting staples, or advancing a knife.
15. The method according to paragraph 13, further comprising:
   displaying a prompt indicating activation of the surgical instrument was prevented.
16. The method according to paragraph 9, further comprising:
   automatically switching from the low visibility mode to a default visibility imaging mode in response to the user input to move the surgical instrument while the surgical instrument is in the first operational mode.
17. A method for controlling a surgical robotic system, the method comprising:
   capturing images using a laparoscopic camera;
   processing the images at an image processing device using a low visibility mode;
   controlling a surgical robotic instrument in a first operational mode while the low visibility mode is active during which the surgical robotic instrument is prevented from being activated; and
   controlling the surgical robotic instrument in a second operational mode while the low visibility mode is not active, during which the surgical robotic instrument is movable and activatable to perform at least one function.
18. The method according to paragraph 17, further comprising:
   receiving user input to move the surgical robotic instrument at a surgeon console including a handle controller.
19. The method according to paragraph 17, further comprising:
   capturing white light and near infrared (NIR) light images at the laparoscopic camera, wherein the low visibility mode is a monochromatic NIR mode.
20. The method according to paragraph 17, wherein the at least one function is selected from the group consisting of supplying electrosurgical energy, ejecting staples, and advancing a knife.

## Claims

1. A surgical robotic system (10) comprising:
an instrument drive unit (52);
a surgical instrument (50) actuated by the instrument drive unit;
a laparoscopic camera (51);
an image processing device (56) coupled to the laparoscopic camera and operable in a plurality of imaging modes including a low visibility mode;
a surgeon console (30) including a handle controller (38a, 38b) receiving user input to move the surgical instrument; and
a controller (21a) operating the surgical instrument in a first operational mode while the low visibility mode is active and in a second operational mode while the low visibility mode is not active.

2. The surgical robotic system according to claim 1, wherein the laparoscopic camera is configured to capture white light and near infrared (NIR) light images.

3. The surgical robotic system according to any preceding claim, wherein the low visibility mode is a monochromatic NIR mode.

4. The surgical robotic system according to any preceding claim, further comprising:
a display screen (32, 34) configured to display a video captured by the laparoscopic camera and processed by the image processing device based on a selected imaging mode of the plurality of imaging modes.

5. The surgical robotic system according to claim 4, wherein while the surgical instrument is in the first operational mode the controller is configured to prevent activation of the surgical instrument.

6. The surgical robotic system according to claim 5, wherein the activation of the surgical instrument includes at least one of supplying electrosurgical energy, ejecting staples, or advancing a knife.

7. The surgical robotic system according to claim 5, wherein the controller is further configured to output a prompt (508) indicating activation of the surgical instrument was prevented.

8. The surgical robotic system according to any preceding claim, wherein while the surgical instrument is in the first operational mode, the controller is configured to switch from the low visibility mode to a default visibility imaging mode in response to the user input to move the surgical instrument.
